# EUROPEAN PATENT SPECIFICATION

(11) **EP 0 392 666 B1**
(45) Date of publication and mention of the grant of the patent: **20.07.1994**
(21) Application number: 90302722.5
(22) Date of filing: 14.03.1990
(51) Int. Cl.: A61K 31/13, A61K 31/315, A61K 31/28

(54) **Anti-HIV compositions**
Anti-HIV Zusammensetzungen
Compositions anti-HIV

(30) Priority: 17.03.1989 GB 8906190
(43) Date of publication of application: 17.10.1990
(73) Proprietor: JOHNSON MATTHEY PUBLIC LIMITED COMPANY, London, EC1N 8JP (GB)
(72) Inventor: Murrer, Barry Anthony, Reading, RG1 5SB (GB)
(74) Representative: Wishart, Ian Carmichael

(56) References cited:
- WO-A-87/02246
- US-A- 3 888 877
- US-A- 4 156 683
- DIALOG INFORMATION SERVICES, file 155, medline, accession no. 06331252; M. ATHAR et al.: "Evaluation of chelating drugs on the toxicity, excretion, and distribution of nickel in poisoned rats", & FUNDAM APPL. TOXICOL. JULY 1987, 9(1), P 26-33
- DIALOG INFORMATION SERVICES, file 155, medline, accession no. 06819283; M. MISRA et al.: "Alleviation of nickel-induced biochemical alterations by chelating agents", & FUNDAM APPL. TOXICOL. AUGUSTUS 1988, 11(2), P 285-92
- JAPAN. J. PHARMACOL., vol. 32, no. 2, April 1982, pages 394-396; H. WATANABE et al.: "Cardiac stimulating effects of macrocyclic polyamines"
- BIOCHEM. J., vol. 245, no. 3, 1987, pages 641-647; E. ROWATT et al.: "The inhibition of infectivity of bacteriophage phiX174 by high-valency metal cations and cyclic polyamines"

## Description

This invention concerns improvements in chemical compounds, especially in the form of pharmaceutical compositions. More particularly it concerns compositions and compounds having activity in in vitro tests on Human Immunodeficiency Virus-infected cells.

The disease known as Acquired Immune Deficiency Syndrome (AIDS) caused by infection by HIV has attracted immense research effort because of the effects of the disease on infected individuals and the dangers of the disease spreading to a wider section of the population. In general, although various chemotherapeutic treatments have been advocated, and some compounds have emerged as a potential basis for treatment, there is still a need for alternatives. In particular, most treatments such as the compound known as AZT have a high toxicity to cells, and it would be desirable to find compounds which are less toxic.

We have found a group of compounds which show interesting properties in in vitro screens of human cells challenged with HIV-1 and/or HIV-2, and are therefore indicated as having potential for the treatment of AIDS and AIDS Related Complex. Accordingly, the present invention proposes the use of compounds as defined below, in pharmaceutical compositions for treating HIV-infected patients. The invention further provides compositions, especially pharmaceutical compositions comprising such a compound in combination or association with a carrier, for example a pharmaceutically acceptable diluent or excipient, for the treatment of viral infections, especially for HIV-infected patients. The invention further provides a process for the preparation of a pharmaceutical composition for the treatment of a patient, comprising the combination of a compound as defined below with a pharmaceutically acceptable diluent or excipient, and formulating said composition into a form suitable for administration to said patient. The invention may also be defined as the use of such a compound for the manufacture of a medicament for the treatment of patients especially HIV-infected patients. It is to be understood that treatment includes prophylactic treatment of patients at risk, in view of the protective properties observed. Anti-viral activity has not been previously reported for the compounds.

Cyclam, described in more detail below, and many of its cyclic amine analogues are generally known and are commercially available. They have been proposed as therapeutic agents for a variety of uses, particularly to remove metals from the system of poisoned mammals and in combination with metals to act as magnetic resonance contrast agents, but it is not believed that there is any commercial use of these compounds. It is also suggested in USP 4,156,683 and GBP 1,304,367 that certain crown-ether-type compounds, which are mainly fused cyclic compounds, have an antiviral activity against influenza virus.

It has also been reported (Biochem. J. (1987), 245, 641-647) that cyclic polyamines act to inhibit the infectivity of a specific bacteriophage ØX174. The most active compound was a hexa-azacyclo-octadecane, with the second most active compound being a triazacyclodecane. The bacteriophages are quite distinct from viruses that infect mammalian cells.

According to the present invention, an active ingredient is provided for anti-HIV use, which is a cyclic amine of 12 to 16 ring members and having 4 amino nitrogen atoms in the ring, or a complex thereof with a non-toxic labile metal atom or complex, or a compound which is a protected form of said amine or complex.

Preferably, the cyclic amine has 13 to 16 ring members.

When considering as active ingredients, complexes of the cyclic amines, it is to be understood that "non-toxic" complexing agents are to be considered with respect to the prognosis for the patient without chemotherapy. Thus complexes with zinc and nickel may be preferred; copper and rhodium for example are not especially suitable.

The invention also includes what are termed "prodrugs", that is protected forms of the cyclic amines or complexes which release the cyclic amine or complex after administration to a patient. For example, the cyclic amine or complex may carry a protective group which is split off by hydrolysis in body fluids, eg in the bloodstream, thus releasing active cyclic amine or complex.

The invention also includes, as would be clear to the skilled man, non-toxic acid addition salts of the cyclic amines etc.

Particular active ingredients are illustrated hereinafter.

The compounds of the invention were tested in a screen by the MTT method (J. Virol. Methods 120: 309-321 [1988]). MT-4 cells (2.5 x 10⁴/well) were infected with HIV-1 (HTLV-IIIB) or HIV-2 (LAV-2 ROD) at a concentration of 100 CCID₅₀ and incubated in the presence of various concentrations of the test compounds, which were added immediately after infection with the virus. After 5 days culture at 37°C in a CO₂ incubator, the number of viable cells was assessed by the MTT (tetrazolium) method. Antiviral activity and cytotoxicity of the compounds are expressed in the Table below as ED₅₀ (»g/ml) and CD₅₀ (»g/ml), respectively. The potential therapeutic usefulness was assessed by calculating a Selectivity Index (SI) corresponding to the ratio of CD₅₀ to ED₅₀. A control test was performed using the known anti-HIV treatment AZT.

**TABLE**

| Compound | HIV-1 | | | HIV-2 | | |
|---|---|---|---|---|---|---|
| | CD₅₀ | ED₅₀ | SI | CD₅₀ | ED₅₀ | SI |
| A | 146.5 | 56.5 | 3 | 258.1 | 22.6 | 11 |
| B | 459.6 | 30.3 | 15 | 487.4 | 40.4 | 12 |
| C | 334.2 | 18.8 | 18 | 340.8 | >500 | <1 |
| D | 11.2 | 0.3 | 37 | 20.5 | >40 | <1 |
| E | 194.2 | 6.96 | 28 | 218.3 | >1000.0 | <1 |
| F | 46.9 | 10.6 | 4 | 46.9 | >100.0 | <1 |
| AZT | >1 | <0.008 | >125 | ND | ND | ND |
| X | 75.3 | >250 | <0.3 | ND | ND | ND |
| Y | >250 | >250 | ∼1 | ND | ND | ND |
| Note: ND = Not Determined Compound A is cyclam B is 1,4,8,12-tetrazacyclopentadecane C is 1,4,8,13-tetrazacyclohexadecane. 4HCl D is 1,5,9,13-tetrazacyclohexadecane E is Zn complex of 1,4,8,12-tetrazacyclopentadecane (Cl⁻) F is Zn complex of 1,5,9,13-tetrazacyclohexadecane (Cl⁻) X is [Cu cyclam]Cl₂ Y is c-[Rh cyclam (NO₂)₂]Cl | | | | | | |

It will be seen from the above results that the compounds of the invention exhibit activity against HIV in cells, and their in vitro toxicity is less than that of AZT.

The metals which are relatively less labile in complexes with cyclam, such as copper and rhodium in the test results above, and cobalt, demonstrate a significantly lower selectivity, because of their higher ED₅₀.

In this field of study, it is considered that any compound exhibiting a Selectivity Index of greater than 5 has the potential for further study and development.

The active compounds as defined may be administered in the form of pharmaceutical compositions formulated according to well known principles and incorporating the compound, preferably in unit dose form, in combination with a pharmaceutically acceptable diluent or excipient. Such compositions may be in the form of solutions or suspensions for injection, or irrigation or be in capsule, tablet, dragee, or other solid composition or as a solution or suspension for oral administration or formulated into pessaries or suppositories or sustained release forms of any of the above or for implantation. Suitable diluents, carriers, excipients and other components of such compositions are known. It may be desirable also to formulate a composition for topical administration such as an ointment or cream. The compounds of the invention may be used, in the form of a composition or alone, and possibly carried on a finely divided support, as a coating on devices or articles which in use contact body fluids, to discourage transmission of viral infections. Examples of devices or articles to be considered in this aspect of the invention are surgical devices and gloves and contraceptives such as condoms, and other items, appliances, wound dressings and coverings, implements etc.

The pharmaceutical compositions according to the invention may be in the form of unit dosages of the active compound determined in accordance with conventional pharmacological methods, suitably to provide active compounds in the dosage range in humans of from 0.1 to 100mg/kg body weight per day, in a single dose or in a number of smaller doses. Preferred dosage ranges are 1 to 30mg/kg body weight per day. Other active compounds may be used in the compositions or such active compounds or supplemental therapy may be included in a course of treatment.

## Claims

1. The use of a cyclic amine of 12 to 16 ring members and having 4 amino groups in the ring, or a complex thereof with a non-toxic labile metal atom or complex, or a prodrug thereof, for the preparation of a medicament for the treatment of patients having, or at risk from, HIV infection.

2. A use according to claim 1, wherein the cyclic amine has from 13 to 16 ring members.

3. The use of a cyclic amine as defined in claim 1 or 2 for the preparation of a coating on a device or article which in use contacts body fluids, for the prevention of transmission of HIV infection.

## Patentansprüche

1. Verwendung eines cyclischen Amins mit 12 bis 16 Ringgliedern und 4 Aminogruppen im Ring oder eines Komplexes davon mit einem nicht-toxischen labilen Metallatom oder Komplex oder eines Pro-Arzneimittels davon zur Herstellung eines Arzneimittels zur Behandlung von Patienten mit einer HIV-Infektion oder dem Risiko für eine HIV-Infektion.

2. Verwendung nach Anspruch 1, worin das cyclische Amin 13 bis 16 Ringglieder hat.

3. Verwendung eines cyclischen Amins nach Anspruch 1 oder 2 zur Herstellung einer Beschichtung auf einer Vorrichtung oder einem Gegenstand, der bei Verwendung mit Körperflüssigkeiten in Kontakt steht, zur Verhütung der Übertragung der HIV-Infektion.

## Revendications

1. Utilisation d'une amine cyclique dodécagonale à hexadécagonale (comportant 12 à 16 chaînons du cycle) et ayant quatre groupes amino dans le cycle, ou d'un complexe de cette amine avec un atome ou un complexe de métal labile et non toxique, ou un précurseur d'un tel produit, pour la préparation d'un médicament destiné au traitement de patients présentant, ou risquant de présenter, une infection par le virus de l'immunodéficience humaine (HIV ou SIDA).

2. Utilisation selon la revendication 1, dans laquelle l'amine cyclique comporte 13 à 16 chaînons du cycle (cycles tridécagonal à hexadécagonal).

3. Utilisation d'une amine cyclique telle que définie à la revendication 1 ou 2 pour la préparation d'un revêtement sur un dispositif ou un article qui, quand on l'utilise, vient en contact avec des fluides corporels, pour la prévention de la transmission de l'infection par le virus du SIDA (par le virus HIV).
